# EUROPÄISCHE PATENTSCHRIFT

(11) **EP 0 597 006 B1**
(45) Veröffentlichungstag und Bekanntmachung des Hinweises auf die Patenterteilung: **25.11.1998**
(21) Anmeldenummer: 92916948.0
(22) Anmeldetag: 03.08.1992
(51) Int. Cl.: C12Q 1/68

(54) **NEUES VERFAHREN ZUR SEQUENZIERUNG VON NUKLEINSÄUREN**
NEW PROCESS FOR SEQUENCING NUCLEIC ACIDS
PROCEDE NOUVEAU DE SEQUENCAGE D'ACIDES NUCLEIQUES

(30) Priorität: 02.08.1991 DE 4125745; 29.04.1992 DE 4214112
(43) Veröffentlichungstag der Anmeldung: 18.05.1994
(73) Patentinhaber: Europäisches Laboratorium für Molekularbiologie, D-69117 Heidelberg (DE)
(72) Erfinder: ANSORGE, Wilhelm, D-6901 Gailberg (DE); VOSS, Hartmut, D-6901 Gailberg (DE)
(74) Vertreter: Huber, Bernhard, Dipl.-Chem.
(86) Internationale Anmeldenummer: EP9201756
(87) Internationale Veröffentlichungsnummer: WO9303180

(56) Entgegenhaltungen:
- EP-A- 0 309 969
- EP-A- 0 371 437
- EP-A- 0 409 078
- WO-A-90/03442
- WO-A-90/04649
- DE-A- 3 839 397
- DE-A- 3 841 565

## Beschreibung

Die vorliegende Erfindung betrifft ein Verfahren zur nicht-radioaktiven Sequenzierung von Nukleinsäuren.

Allgemein sind für die DNA-Sequenzierung zwei Methoden bekannt, nämlich das chemische Abbauverfahren nach Maxam und Gilbert (A.M. Maxam und W. Gilbert, Proc.Natl.Acad.Sci. USA 74 (1977), 560 und A.M. Maxam und W. Gilbert, Methods in Enzymol. 65 (1980), 499) und die enzymatische Kettenabbruchmethode (F. Sanger et al., Proc.Natl.Acad.Sci. USA 74 (1977), 5463-5467).

Ein direktes Sequenzierverfahren wird in WO90/04649 beschrieben. Diese Methode bedient sich der Polymerasekettenreaktion und Deoxynukleosid-thio-triphosphaten, die als Spaltstellen für eine auf die Polymerasekettenreaktion folgende chemische oder enzymatische Reaktion dienen. Weiter dient die Thiogruppe zur Endmarkierung mit radioaktiven, nicht-radioaktiven oder enzymatischen Gruppen zum Nachweis der zu sequenzierenden Nukleinsäurefragmente.

Bei der Maxam-Gilbert-Methode werden markierte DNA-Moleküle chemisch in basenspezifischer Weise modifiziert, ein partieller Strangabbruch bewirkt und die so erhaltenen Fragmente nach Größe aufgetrennt und die Sequenz anhand der Markierung bestimmt.

Bei der Methode nach Sanger werden, ausgehend von einer DNA-Matrize, viele verschieden lange markierte Nukleinsäurefragmente durch enzymatische Elongation bzw. Extension eines synthetischen Oligonukleotidprimers mit Hilfe von Polymerase und einer Mischung von Deoxyribonukleosidtriphosphaten und Kettenabbruchmolekülen, insbesondere Dideoxyribonukleosidtriphosphaten hergestellt. Hierbei wird üblicherweise jeweils in vier Ansätzen eine Mischung eines bestimmten Deoxyribonukleosidtriphosphats (dNTP) und eines entsprechenden Dideoxyribonukleosidtriphosphats (ddNTP) zusammen mit den drei anderen Deoxyribonukleosidtriphosphaten eingesetzt. Auf diese Weise wird ein statistischer Einbau der Kettenabbruchmoleküle in die wachsenden Nukleinsäureketten erreicht, wobei nach dem Einbau eines Kettenabbruchmoleküls die DNA-Kette aufgrund des Fehlens einer freien 3'-OH-Gruppe nicht mehr weiter wachsen kann. Es entsteht daher eine Vielzahl von unterschiedlich langen DNA-Fragmenten, die statistisch gesehen, mindestens an jeder möglichen Einbaustelle ein Kettenabbruchmolekül enthalten und dort enden. Diese vier Ansätze mit den jeweils aufgrund des Einbaus von Kettenabbruchmolekülen spezifisch an einer Base endenden Fragmenten werden nach ihrer Länge, z.B. durch Polyacrylamid-Gelelektrophorese, üblicherweise in vier verschiedenen Spuren aufgetrennt und die Sequenz anhand einer Markierung dieser Nukleinsäurefragmente ermittelt.

Bis etwa 1986 wurde die DNA-Sequenzierung unter Verwendung von radioaktiven (³²P- oder ³⁵S)-Markierungen durchgeführt. Die gelelektrophoretische Auftrennung der Fragmente und Autoradiographie der Nukleotidsequenz erfolgte entweder manuell oder halbautomatisch. Heute wird die DNA-Sequenzierung mit automatisierten Systemen durchgeführt, bei denen eine nicht-radioaktive Markierung, insbesondere eine Fluorszenzmarkierung verwendet wird (L.M. Smith et al., Nature 321 (1986), 674-679; W. Ansorge et al., J.Biochem. Biophys.Meth. 13 (1986), 315-323). Bei diesen automatisierten Systemen wird die Nukleotidsequenz direkt während der Auftrennung der markierten Fragmente gelesen und direkt in einen Computer eingegeben.

Gegenwärtig stehen zwei verschiedene nicht-radioaktive Markierungssysteme für die DNA-Sequenzierung nach der Kettenabbruchmethode zur Verfügung. Eine Möglichkeit der Markierung von Nukleinsäurefragmenten besteht darin, einen markierten Oligonukleotidprimer für die Extensionsreaktion einzusetzen, so daß die Nukleinsäurekette an ihrem 5'-Ende eine Markierung trägt. Die andere Möglichkeit besteht darin, markierte Terminatoren, d.h. Kettenabbruchmoleküle zu verwenden, so daß die Nukleinsäurekette an ihrem 3'-Ende eine Markierung trägt. Eine Arbeit von Prober et al. (Science 238 (1987), 336-341) beschreibt ein Sequenzierungsverfahren, bei dem die vier Ketten-terminierenden ddNTPs jeweils mit unterschiedlichen Fluoreszenzfarbstoffen gekoppelt sind, so daß alle vier Sequenzieransätze auf einer einzigen Gelspur aufgetrennt werden können. Ein ähnliches System mit 4 unterschiedlich markierten Primern ist in der GB-A 2155176 offenbart.

DE-OS 38 41 565 beschreibt die Verwendung von Fluoreszenzfarbstoffen, die über einen Linker mit den Nukleinsäurefragmenten verbunden sind. Die Markierung mit dem Fluoreszenzfarbstoff befindet sich dabei entweder an der 5'-Phosphatgruppe des Primers oder am 3'-Ende des Nukleinsäurefragments an der Purin- oder Pyrimidinbase. Durch Einsetzen von zwei der Dideoxynukleosidtriphosphate in unterschiedlichen Mengen zusammen in einem Ansatz ist die Unterscheidung der Fragmente über die unterschiedliche Intensität des Markierungssignals der Banden im Gel oder in einem anderen Trennverfahren möglich. EP 0 409 078 offenbart auch die Verwendung von endständig markierten Oligonukleotidprimern oder markierten Deoxy- bzw. Dideoxynukleotidtriphosphaten. Zur Erhöhung der Signalintensitäten werden ein Hitzedenaturierungsschritt, ein Annealingschritt und der Nukleinsäuresyntheseschritt mit teilweisem Kettenabbruch 10 bis 60 x wiederholt und anschließend das erhaltene Deoxynukleinsäurefragmentgemisch der Länge nach auftrennt und aus dem Fragmentspektrum die Sequenz bestimmt. Aber auch für andere Anwendungen, wie z.B. beschrieben in EP 0 309 969 zum Genmapping, werden Fluoreszenzfarbstoffe zur Endmarkierung von Nukleinsäurefragmenten verwendet. Hierbei wird nach dem Verdau der Nukleinsäuren mit einer Restriktionsendonuklease der "Reporter" an der spezifisch geschnittenen Base angebracht.

Auch bei der Maxam-Gilbert-Methode werden mittlerweile Fluoreszenzfarbstoffe zur Endmarkierung von Nukleinsäurefragmenten verwendet. DE-OS 38 39 397 offenbart eine Sequenziermethode nach Maxam und Gilbert unter Verwendung von Fluoreszenzfarbstoffen, in der die Nachteile der Verwendung von Radioaktivität vermieden werden.

Bei den oben genannten Methoden wird aber nur eine einzige Markierung in die zu bestimmende Nukleinsäurekette eingeführt, was oft zu Sensitivitätsproblemen führen kann. Ein weiterer Nachteil bei der Verwendung von farbstoffmarkierten Oligonukleotidprimern, insbesondere bei einer "Walking Primer"-Strategie ist, daß für jede Sequenzierungsreaktion unterschiedliche Primer verwendet werden müssen, wobei die Herstellung von markierten Primern aber sehr kostspielig oder/und aufwendig ist. Überdies wird durch die Verwendung markierter Primer manchmal die Hybridisierung des Primers mit der Nukleinsäurematrize behindert, was zu ungenauen Sequenzierungsresultaten führen kann.

Auch die Verwendung von farbstoffmarkierten Terminatoren, insbesondere Dideoxyribonukleosidtriphosphaten, hat sich als nicht befriedigend erwiesen. Diese markierten Terminatoren werden von der Polymerase nur schlecht als Substrate akzeptiert, so daß sie dem Reaktionsansatz in großer Konzentration (millimolarer Bereich) und in hochgereinigter Form (quantizative Abtrennung von unmarkierten Molekülen) zugesetzt werden müssen. Dies führt dazu, daß die Verwendung von markierten Terminatoren sehr kostspielig ist. Überdies werden schlechtere Ergebnisse als z.B. bei Verwendung von markierten Primern erhalten.

Aufgabe der vorliegenden Erfindug war es somit, ein Verfahren zur Sequenzierung von Nukleinsäuren bereitzustellen, bei dem die Nachteile des Standes der Technik zumindest teilweise beseitigt sind.

Die erfindungsgemäße Aufgabe wird gelöst durch ein Verfahren zur Sequenzierung von Nukleinsäuren durch
(a) Erzeugung eines Gemisches von markierten Nukleinsäurefragmenten mit unterschiedlicher Länge,
(b) Auftrennung der markierten Nukleinsäurefragmente nach Größe und
(c) Bestimmung der Nukleinsäuresequenz über die Markierung der einzelnen Fragmente,
welches dadurch gekennzeichnet ist, daß man Nukleinsäurefragmente bestimmt, in die durch Einbau mit mindestens einem Deoxyribonukleosidtriphosphat, das eine nicht-radioaktive Markierungsgruppe trägt, eine innere Markierung eingeführt worden ist, wobei die Einführung der inneren Markierung in die Nukleinsäurefragmente in Abwesenheit von Kettenabbruchmolekülen stattfindet.

Die Sequenzierung nach dem erfindungsgemäßen Verfahren kann sowohl durch die chemische Abbaumethode nach Maxam und Gilbert als auch durch die enzymatische Kettenabbruchmethode nach Sanger erfolgen. In beiden Fällen erhält man ein Gemisch von nicht-radioaktiv markierten Nukleinsäurefragmenten mit unterschiedlicher Länge, die als "innere" Markierung mindestens ein Deoxyribonukleosidmonophosphat enthalten, das mit einer nicht-radioaktiven Markierungsgruppe versehen ist. Allgemein ist festzustellen, daß sich das erfindungsgemäße Verfahren auf einfache Weise bei allen bekannten DNA-Sequenzierungsprotokollen (z.B. auch bei Festphasen-Sequenzierungen) anwenden läßt.

Als nicht-radioaktive Markierungsgruppen sind beispielsweise Metalle, magnetische Markierungsgruppen, die durch Kernresonanz oder durch einen supraleitenden quanten-interferometrischen Detektor (SQUID) nachweisbar sind, Phosphoreszenzoder Fluoreszenzfarbstoffe geeignet. Besonders bevorzugt sind Fluoreszenzfarbstoffe, beispielsweise Rhodamin, Texasrot, Phycoerythrin oder Fluorescein und seine Derivate. Insbesondere sind solche Fluoreszenzfarbstoffe bevorzugt, die eine Absorption in einem Bereich von 600 bis 800 nm zeigen. Beispiele für eine Vielzahl geeigneter Farbstoffe sind im Katalog der Firma Molecular Probes zu finden.

Bei den markierten Deoxyribonukleosidtriphosphaten ist die Markierung vorzugsweise über einen Linker an die C₅-Position von Pyrimidinbasen (Uracil, Thymin oder Cytosin) oder an die N₇ oder C₈-Position von Purinbasen (Adenin, Guanosin, Hypoxanthin) oder an die C₇-Position von Purinbasen (7-Deazaguanidin) gekoppelt. Die Anzahl der Atome in der Kette des Linkers ist im allgemeinen 1 bis 50, vorzugsweise 4 bis 24 und besonders bevorzugt 10 bis 18.

Die Verwendung von markierten dNTPs wurde bereits in der DE-OS 38 41 565 als wünschenswert vorgeschlagen. Zu diesem Zeitpunkt waren aber weder geeignete chemische Substanzen verfügbar, noch war bekannt, ob diese Substanzen sich bei der Sequenzierungsreaktion, insbesondere bei einer automatisierten Sequenzierungsreaktion als geeignete Substrate erweisen.

Überraschenderweise hat sich Fluorescein-markiertes dUTP (Fluorescein-12-2'-Deoxyuridin-5'-triphosphat bzw. Fluorescein-12-dUTP), das kommerziell von Boehringer Mannheim (Katalog Nr. 1373242) erhältlich ist, als besonders geeignet erwiesen. Fluorescein-12-dUTP kann bei dem erfindungsgemäßen Verfahren anstelle von dTTP oder zusammen mit dTTP verwendet werden. Weiterhin haben sich auch Fluorescein-12-2'-dCTP, Fluorescein-15-2'-dGTP und insbesondere Fluorescein-15-2'-dATP als hervorragend geeignete markierte dNTPs erwiesen.

Bei dem erfindungsgemäßen Verfahren können pro Ansatz ein einziges markiertes dNTP oder auch mehrere markierte dNTPs nebeneinander eingesetzt werden. Durch gleichzeitige Verwendung mehrerer markierter dNTPs kann die Anzahl der markierten Nukleotide in einer Kette stark erhöht werden, was z.B. günstig ist, wenn das zu sequenzierende Ausgangsmaterial nur in sehr geringer Konzentration zur Verfügung steht.

Wird das erfindungsgemäße Verfahren nach der chemischen Abbaumethode von Maxam und Gilbert durchgeführt, so gibt es verschiedene Möglichkeiten die Markierung in die zu sequenzierende Nukleinsäure einzuführen.

Einerseits kann etwa die Markierung über enzymatische Extension eines Oligonukleotidprimers in Gegenwart einer Polymerase, der zu sequenzierenden Nukleinsäure als Matrize und aller vier Deoxyribonukleosidtriphosphate, von denen mindestens eines eine Markierungsgruppe enthält, eingeführt werden. Dabei entsteht durch Verlängerung des Primers ein zur sequenzierenden Nukleinsäure komplementärer Strang, der Markierungsgruppen trägt, und der anschließend auf übliche Weise, d.h. durch partielle basenspezifische Spaltung mittels chemischer Methoden und anschließende Auftrennung des resultierenden Fragmentgemisches sequenziert werden kann.

Beispiele für weitere bevorzugte Ausführungsformen des Maxam-Gilbert-Verfahrens sind in der DE-OS 38 39 397 offenbart, auf deren Inhalt hier Bezug genommen wird.

Die erfindungsgemäße Sequenzierung erfolgt vorzugsweise durch die enzymatische Kettenabbruchmethode nach Sanger. Bevorzugt ist ein Verfahren zur nicht-radioaktiven Sequenzierung von Nukleinsäuren durch
(a) enzymatische Extension eines Oligonukleotidprimers in Gegenwart von Polymerase, Deoxyribonukleosidtriphosphaten, Kettenabbruchmolekülen und der zu sequenzierenden Nukleinsäure als Matrize in einem oder mehreren Ansätzen, wobei ein Gemisch von markierten Nukleinsäurefragmenten mit unterschiedlicher Länge gebildet werden,
(b) Auftrennung der markierten Nukleinsäurefragmente nach Größe und
(c) Bestimmung der Nukleinsäuresequenz über die Markierung der einzelnen Fragmente,
welches dadurch gekennzeichnet ist, daß man die Elongation des Oligonukleotidprimers in Gegenwart von mindestens einem Deoxyribonukleosidtriphosphat, das eine nicht-radioaktive Markierungsgruppe trägt, durchführt, wobei die Einführung der inneren Markierung in die Nukleinsäurefragmente in Abwesenheit von Kettenabbruchmolekülen stattfindet.

Überraschenderweise wurde festgestellt, daß nicht-radioaktiv markierte Deoxyribonukleosidtriphosphate, insbesondere mit Fluoreszenzfarbstoffen markierte Deoxyribonukleosidtriphosphate, bei einer Sequenzierungsreaktion in ausreichendem Umfang von der Polymerase als Substrat akzeptiert werden. Bei Einsatz von Fluorescein-12-dUTP, Fluorescein-12-dCTP, Fluorescein-15-dATP und Fluorescein-15-dGTP als nicht-radioaktiv markierte Deoxyribonukleosidtriphosphate und jeweils den anderen nicht markierten Deoxyribonukleosidtriphosphaten auf einem automatischen DNA-Sequenziergerät (A.L.F. DNA-Sequencer von Pharmacia-LKB) wurden hervorragende Resultate erhalten.

Im Gegensatz zu den Verfahren gemäß dem Stand der Technik können bei dem erfindungsgemäßen Verfahren gleich mehrere markierte Nukleotide in ein einziges DNA-Fragment eingebaut werden, wodurch sich die Signalstäke und damit die Sensitivität der Bestimmung um eine Größenordnung oder mehr erhöhen.

Ferner ermöglicht die hohe Signal stärke einen sensitiven Nachweis von Fluoreszenzbanden in einem automatischen Sequenziergerät, sogar wenn sie sehr schnell durch den als Detektor verwendeten Laserstrahl wandern, so daß eine Durchführung von DNA-Sequenzierungsreaktionen, die mit sehr hoher Geschwindigkeit, bis zu mehreren Tausend Basen pro Stunde pro Klon arbeiten, durch Verwendung hoher Spannungen, z.B. in ultradünnen Plattengelen oder in ultradünnen Kapillaren ermöglicht wird.

Ein Vorteil des erfindungsgemäßen Verfahrens ist seine Einfachheit, da keine markierten Primer bzw. keine markierten Kettenabbruchmoleküle benötigt werden. Dies ist insbesondere für Sequenzierprojekte in großem Maßstab, wie die Sequenzierung des menschlichen Genoms oder anderer Genomprojekte bedeutsam. Bei Verwendung von markierten dNTPs bei der Sequenzierungsreaktion können Standardprimer verwendet werden, so daß eine zeit- oder/und kostenintensive Herstellung von markierten Oligonukleotidprimern vermieden werden kann. Andererseits können, sofern gewünscht, natürlich auch markierte Primer verwendet werden.

Ein weiterer Vorteil des erfindungsgemäßen Verfahrens besteht darin, daß die Auflösung der Sequenzleiter insbesondere bei größeren Länge überraschenderweise gegenüber der aus dem Stand der Technik bekannten Verwendung von markierten Primern verbessert wird. Dies gilt insbesondere bei einer Verfahrensführung, wo die Markierung und die Extension in zwei getrennten Schritten erfolgen (siehe Beispiele 2 und 3) und bei Verwendung von Fluorescein-dATP oder Fluorescein-dUTP als Markierung.

Das erfindungsgemäße Verfahren zeichnet sich dadurch aus, daß man einen Oligonukleotidprimer ohne Markierungsgruppe, insbesondere einen am 5'-Ende unmodifizierten Oligonukleotidprimer einsetzen kann. Andererseits ist es aber auch möglich, in Kombination mit den markierten dNTPs einen Oligonukleotidprimer zu verwenden, der an seinem 5'-Ende mit einem Farbstoff oder/und einer zu einer Affinitätsbindung fähigen Gruppe modifiziert ist. So ist z.B. eine Doppelmarkierung möglich, d.h. man verwendet einen Farbstoff-markierten Oligonukleotidprimer, der eine andere Fluoreszenzwellenlänge als die bei dem entsprechenden Ansatz verwendeten markierten dNTPs aufweist. Ferner kann der Oligonukleotidprimer an seinem 5'-Ende biotinyliert sein, wodurch z.B. die Bindung an eine mit Streptavidin beschichtete Festphase möglich ist. Andere Beispiele für Gruppen, die zu einer Affinitätsbindung fähig sind, sind Haptene (z.B. Digoxigenin oder Digoxin), die mit einem für das entsprechende Hapten spezifischen Antikörper bindefähig sind.

Als Kettenabbruchmoleküle für das erfindungsgemäße Verfahren werden zweckmäßigerweise Deoxyribonukleosidtriphosphate verwendet, die an der 3'-Position der Deoxyribose so modifiziert sind, daß sie keine freie OH-Gruppe aufweisen, und aber dennoch von der Polymerase als Substrat akzeptiert werden. Beispiele für solche Kettenabbruchmoleküle sind etwa 3'-Fluor-, 3-O-Alkyl- oder 3'-H-modifizierte Deoxyribonukleotide. Vorzugsweise werden als Kettenabbruchmoleküle 3'-H-modifizierte Deoxyribonukleotide, d.h. Dideoxyribonukleosidtriphosphate (ddNTPs) verwendet. Vorzugweise arbeitet man bei dem erfindungsgemäßen Verfahren mit unmarkierten Kettenabbruchmolekülen, es ist jedoch auch möglich, markierte Kettenabbruchmoleküle, wie sie dem Fachmann bekannt sind, zu verwenden.

Erfindungsgemäß werden zur enzymatischen Extension eines Oligonukleotidprimers bzw. zur Auffüllung von Restriktionsfragmenten vorzugsweise das Klenow-Fragment der E.coli-DNA-Polymerase I, modifizierte oder unmodifizierte T7-DNA-Polymerase, T4-DNA-Polymerase, Taq-DNA-Polymerase, Bst-DNA-Polymerase oder Reverse Transkriptase verwendet. Von diesen Polymerasen sind die T7-DNA-Polymerase und die thermostabilen Taq- und Bst-DNA-Polymerasen besonders bevorzugt. Überraschenderweise werden die nicht-radioaktiv markierten dNTPs von diesen Enzymen bei einer Sequenzierungsreaktion als Substrate akzeptiert.

Die Auftrennung der markierten Nukleinsäurefragmente kann bei dem erfindungsgemäßen Verfahren nach allen in der Technik bekannten Methoden, z.B. durch verschiedene elektrophoretische (z.B. Polyacrylamid-Gelelektrophorese) oder chromatographische (z.B. HPLC) Verfahren erfolgen, wobei eine gelelektrophoretische Auftrennung bevorzugt ist. Ferner kann die Auftrennung der markierten Nukleinsäuren auf an sich beliebige Weise, d.h. manuell, halbautomatisch oder automatisch erfolgen, wobei jedoch die Verwendung eines automatischen Sequenziergeräts im allgemeinen bevorzugt ist. In diesem Fall kann dann die Auftrennung der markierten Nukleinsäuren in ultradünnen Plattenqelen von 20 bis 200 µm, vorzugsweise 100 µm Dicke (siehe z.B. Stegemann et al., Methods in Mol. and Cell.Biol. 2 (1991), 182-184) oder Kapillaren durchgeführt werden.

Bei Verwendung von Deoxyribonukleosidtriphosphaten, die mit Fluoreszenzfarbstoffen markiert sind, kann die Bestimmung der Markierung nach Auftrennung der einzelnen Nukleinsäurefragmente gemäß ihrer Größe dadurch erfolgen, daß man den Fluoreszenzfarbstoff durch Laser oder andere geeignete Lichtquellen (z.B. LEDs, Fluoreszenzlampen oder Diodenlase) anregt. Derartige Methoden sind dem Fachmann bekannt und werden in kommerziell verwendeten Sequenziergeräten (z.B. A.L.F. Sequencer von Pharmacia) bereits eingesetzt.

Steht für eine Sequenzbestimmung nach dem erfindungsgemäßen Verfahren nur eine sehr geringe Menge der zu sequenzierenden Nukleinsäure zur Verfügung, so kann ein Amplifizierungsschritt durchgeführt werden. Eine Möglichkeit der Amplifizierung besteht darin, einen oder mehrere Zyklen einer Polymerase-Chain-Reaktion (PCR) unter Verwendung von zwei Primern vor der eigentlichen Sequenzierung durchzuführen. Die PCR erfolgt üblicherweise ohne markierte dNTPs. So kann die zu sequenzierende Nukleinsäure vor Durchführung der eigentlichen Sequenzierung amplifiziert werden.

Andererseits kann der Amplifizierungsschritt auch mit Hilfe einer "Thermocycling"-Reaktion erfolgen. Die Thermocycling-Reaktion entspricht einer "normalen" Sequenzierungsreaktion, die allerdings - wie eine PCR - in mehreren Zyklen durchgeführt wird. Der Reaktionsansatz enthält die NukleinsäureMatrize, den Primer, die dNTPs und jeweils entsprechende Kettenabbruchmoleküle sowie eine vorzugsweise thermostabile Polymerase. Auf diese Weise wird pro Zyklus immer eine bestimmte Menge der markierten Nukleinsäurefragmente synthetisiert, wobei in mehreren Zyklen große Mengen an markierten Fragmente erzeugt werden können, so daß die DNA linear und nicht wie bei der PCR exponentiell amplifiziert wird.

Die zu sequenzierende Nukleinsäure kann sowohl in einzelsträngiger als auch in doppelsträngiger Form vorliegen. Es werden gute Ergebnisse erhalten, wenn sich die zu sequenzierende Nukleinsäure auf einem doppelsträngigen DNA-Vektor, z.B. einem Plamsid, Cosmid, Bakteriophagen (Lambda oder P1), einem viralen Vektor oder einem künstlichen Chromosom (künstliches Hefechromosom) befindet.

In einer ersten bevorzugten Ausführungsform des erfindungsgemäßen Verfahrens führt man die enzymatische Elongationsreaktion auf übliche Weise, d.h. in mehreren getrennten Ansätzen durch, wobei man in jedem Ansatz unterschiedliche Kettenabbruchmoleküle, d.h. Terminatoren mit unterschiedlichen Nukleotidbasen, verwendet. Die Kettenabbruchmoleküle können entweder gleich zu Beginn der Reaktion zugesetzt werden oder die Reaktion kann in zwei Schritten, einem Extensionsschritt in Abwesenheit von Kettenabbruchmolekülen und einem Terminationsschritt in Gegenwart des jeweiligen Kettenabbruchmoleküls durchgeführt werden. Verwendet man T7-DNA-Polymerase zur Kettenverlängerung, kann z.B. der Extensionsschritt (ohne ddNTPs) in Gegenwart von Magnesium- und der Terminationsschritt (mit ddNTPs) in Gegenwart von Manganionen durchgeführt werden.

Gemäß dieser Ausführungsform werden vorzugsweise vier parallele Ansätze durchgeführt, wobei in jedem Ansatz ein anderes ddNTP als Kettenabbruchmolekül vorhanden ist. Werden in diesen vier Ansätzen jeweils gleiche Markierungen (ein oder mehrere markierte dNTPs) verwendet, ist eine getrennte Auftrennung der einzelnen Ansätze erforderlich. In einer bevorzugten Variante dieser Ausführungsform kann man zwei (oder mehrere) Sequenzierungsreaktionen mit unterschiedlich markierten dNTPs durchführen, wobei jeweils solche Markierungsgruppen verwendet werden, die nebeneinander nachweisbar sind. So kann man z.B. in einer ersten Reaktion (d.h. in allen 4 Ansätzen dieser Reaktion) Fluorescein-dNTP und in einer zweiten Reaktion Rhodamin-dNTP verwenden. Die Auswertung dieser beiden Reaktionen kann gemeinsam erfolgen, wobei man 2 Ansätze mit jeweils unterschiedlichen Markierungen (d.h. eine Fluorescein- und eine Rhodamin-Markierung) auf eine einzige Gelspur aufträgt und getrennt (durch Messung bei unterschiedlichen Fluoreszenzwellenlängen) bestimmt.

Setzt man andererseits bei einer Reaktion in verschiedenen Ansätzen jeweils unterschiedliche Markierungen ein (z.B. durch Verwendung von Fluoreszenzfarbstoffen mit unterschiedlichen Absorptionswellenlängen), so können auch mehrere oder sogar alle Ansätze einer einzigen Reaktion gemeinsam, z.B. auf einer Gelspur, aufgetrennt werden.

Eine andere bevorzugte Ausführugnsform des erfindungsgemäßen Verfahrens besteht darin, daß man in einem einzigen Ansatz mindestens zwei verschiedene Kettenabbruchmoleküle in unterschiedlichen Mengen zusammen einsetzt, wobei in diesem Falle eine Unterscheidung der Fragmente über die Intensität ihres Markierungssignals getroffen werden kann. Derartige Verfahren sind beispielsweise Gegenstand der DE-OS 38 41 565.

Ein weiterer Gegenstand der vorliegenden Erfindung ist die Verwendung von nicht-radioaktiv markierten Deoxyribonukleosidtriphosphaten bei Verfahren zur Sequenzierung von Nukleinsäuren nach der Abbaumethode von Maxam und Gilbert oder nach der Kettenabbruchmethode von Sanger. Weiterhin werden Deoxyribonukleosidtriphosphate, die eine nicht-radioaktive Markierung tragen, zur inneren Markierung von Nukleinsäurefragmenten bei einem der beschriebenen Verfahren zur Sequenzierung von Nukleinsäuren verwendet, wobei die Einführung der inneren Markierung in die Nukleinsäurefragmente in Abwesenheit von Kettenabbruchmolekülen stattfindet. Die Markierung ist dabei vorzugsweise ein Fluoreszenzfarbstoff, besonders bevorzugt Fluorescein oder ein Derivat davon. Die Markierung ist vorzugsweise über einen Linker mit dem dNTP verknüpft. Die Anzahl der Atome in der Kette des Linkers beträgt im allgemeinen 1 bis 50, vorzugsweise 4 bis 24 und besonders bevorzugt 8 bis 18. Als günstig für ein automatisches Sequenzierverfahren hat sich die Verwendung von Fluorescein-12-dUTP, Fluorescein-12-dCTP, Fluorescein-15-dGTP oder/und Fluorescein-15-dATP erwiesen.

Noch ein weiterer Gegenstand der Erfindung ist die Verwendung eines Reagenzienkits zur Sequenzierung von Nukleinsäuren gemäß den beschriebenen Verfahren, der als Reagenz mindestens ein Deoxyribonukleosidtriphosphat enthält, das mit einer nicht-radioaktiven Markierungsgruppe markiert ist, sowie ferner ein Kettenabbruchmolekül. Das Reagenz kann z.B. in Form einer Lösung, einer Suspension oder eines Lyophilisats sein. Der Reagenzienkit kann als zusätzliche Reagenzien (z.B. Enzym, Primer, Pufferlösungen, nicht markierte NTPs, Terminatoren) enthalten.

Die folgenden Beispiele sollen die Erfindung in Verbindung mit den Figuren 1 bis 3 weiter verdeutlichen.
- Figur 1: zeigt das Ergebnis einer Plasmidsequenzierung mit Fluorescein-12-dUTP als Markierung.
- Figur 2: zeigt das Ergebnis einer Plasmidsequenzierung mit Fluorescein-12-dCTP als Markierung,
- Figur 3: zeigt das Ergebnis einer Plasmidsequenzierung mit Fluorescein-15-dATP als Markierung.

### Beispiel 1

### DNA-Sequenzierung mit einer Fluorescein-12-dUTP-Markierung

Die Sequenzierung wurde mit einem automatischen DNA-Sequenziergerät (A.L.F. DNA-Sequencer von Pharmacia LKB) durchgeführt. Es wurden Versuche mit T7-DNA-Polymerase und Mangan- bzw. Magnesiumpuffer unter Verwendung von einzelsträngigen und doppelsträngigen DNA-Matrizen durchgeführt. Die Nukleotide und Enzyme wurden von Pharmacia PL bezogen. Fluorescein-12-dUTP wurde von Boehringer Mannheim bezogen. Gute Ergebnisse wurden unter Verwendung des folgenden Reaktionsprotokolls erhalten:

### 1. Denaturierung und Annealing

5 µg Plasmid-DNA (hergestellt durch Reinigung über eine Qiagen-Säule) in 6 µl bidest H₂O wurden mit 2 µl Sequenzierprimer (1 µmol/l) vermischt, durch Zugabe von 1 µl 1M NaOH für 3 Minuten bei 70°C denaturiert und mit 1 µl 1M HCl neutralisiert. 2 µl frisch hergestellter 10X Reaktionspuffer (300 mmol/l Tris HCl, pH 7,5, 150 mmol/l DTT, 60 mmol/l MnCl₂, 460 mmol/l DL-Isocitrat) wurden zugegeben und für 15 Minuten bei 37°C inkubiert.

### 2. Extension und Markierung

Zu dem obigen Ansatz wurden 2 µl Markierungsmischung (jeweils 1,5 µmol/1 dATP, dCTP, dGTP und Fluorescein-12-dUTP) zusammen mit 3,5 U T7-DNA-Polymerase gegeben und 10 Minuten bei Raumtemperatur inkubiert.

### 3. Termination

4 µl Aliquots des Reaktionsprodukts wurden zu 3 µl der vier jeweiligen Terminationsmischungen (1 mmol/l dATP, 1 mmol/l dCTP, 1 mmol/l 7-Deaza-dGTP, 1 mmol/l dTTP, jeweils 5 pmol/l ddNTP) gegeben und 5 Minuten bei 37°C inkubiert. Die Reaktionen wurden mit 4 µl deionisiertem Formamid mit 5 mg/ml Dextranblau gestoppt, durch Erhitzen denaturiert und auf das Sequenzgel gegeben.

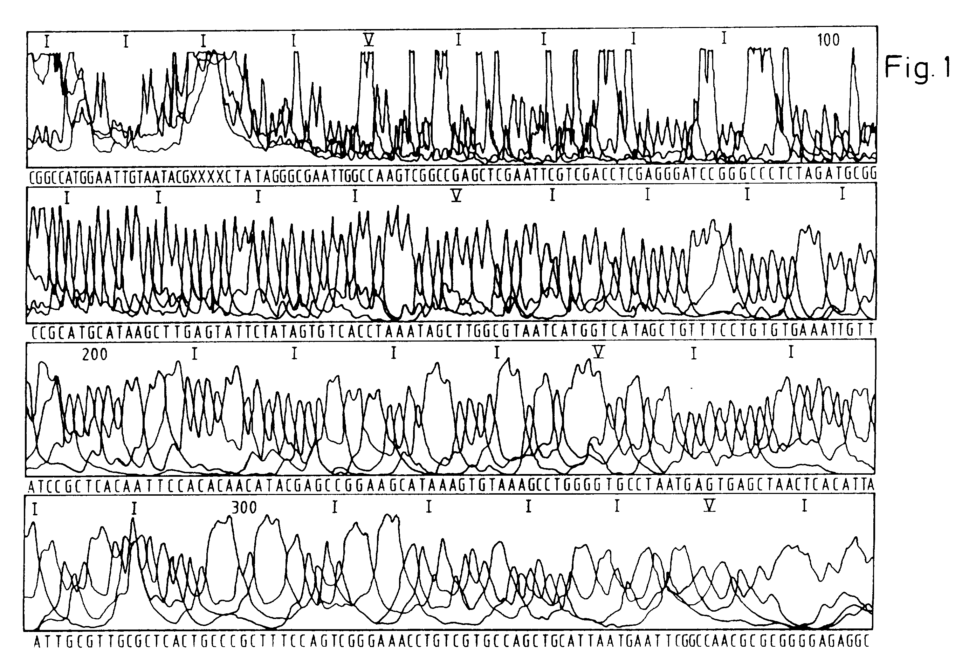

Figur 1 zeigt das Ergebnis einer typischen Plasmidsequenzierung unter Verwendung von Fluorescein-12-dUTP als Markierung. Das Signal beginnt direkt nach dem ersten markierten T-Rest nach der Primingstelle ohne sichtbaren Primerpeak. Die Intensitäten bei multiplen Peaks sind gleich wie in Primerexperimenten mit Endmarkierung. Sowohl Magnesium- als auch Manganpuffer für die T7-DNA-Polymerase haben sich als geeignet erwiesen.

Eine automatisierte DNA-Sequenzierung mit Fluoreszenz-markierten dNTPs weist gegenüber anderen Markierungstechniken verschiedene Vorteile auf.
1. Die Markierungsintensität ist eindeutig stärker als bei bekannten Protokollen. Dabei sind 200 ng ss-DNA zur Sequenzierung ausreichend. Bei bekannten Verfahren wird üblicherweise ca. 1 µg ss-DNA eingesetzt.
2. Jeder beliebige unmarkierte Oligonukleotidprimer (z.B. Primer für Expressionsvektoren) kann direkt verwendet werden. Die "Walking Primer"-Methode wird erleichtert, da keine Markierung von Primern oder eine Reinigung des markierten Produkts erforderlich ist. Da keine Wechselwirkung mit einem Farbstoff am 5'-Ende des Primers auftreten kann, sind kürzere Oligonukleotide (12-15 Basen) zur Verwendung geeignet.
3. Im Vergleich zu Fluoreszenz-markierten ddNTPs ist der Preis der Markierung vernachlässigbar (weniger als 0,05 DM pro Reaktion). Überdies ist keine Entfernung von nicht eingebauter Markierung erforderlich.
4. Das Fluoreszenz-markierte dUTP wird von vielen DNA-Polymerasen (T4, T7, Klenow, Taq, Bst, AMV-Reverse Transkriptase) als Substrat akzeptiert.

### Beispiel 2

Einzelsträngige DNA-Sequenzierung mit Fluorescein-dNTPs unter Verwendung des Pharmacia AutoRead Kits

### 1. Denaturierung und Annealing (in einem Gefäß)

10 µl einzelsträngige Template-DNA (200 ng M13), 2 µl unmarkierter Sequenzierprimer (5 µmol/l) und 2 µl Annealing-Puffer wurden vermischt, 3 Minuten lang auf 65°C erhitzt und auf 37°C abkühlen gelassen.

### 2. Markierung

Zu dem obigen Ansatz wurde 1 µl Markierungsmischung (10 µmol/l markiertes dNTP und jeweils 1 µmol/l der nicht markierten dNTPs) zusammen mit 0,5 µl T7-DNA-Polymerase (3,5 U) gegeben, vermischt und 10 Minuten lange bei 37°C inkubiert.

### 3. Termination

Der obige Ansatz wurde mit 1 µl Extensionspuffer vermischt. Dann wurden 4 µl Aliquots davon zu 4 µl der jeweiligen A-, C-, G- und T-Mixe (3 µl der jeweiligen Terminationsmischung und 1 µl DMSO) gegeben und 5 Minuten lang bei 37°C inkubiert. Die Reaktionen wurden mit jeweils 4 µl Stoplösung gestoppt. Die Ansätz wurden anschließend durch Erhitzen denaturiert und auf das Sequenzgel gegeben.

### Beispiel 3

Doppelsträngige DNA-Sequenzierung mit Fluorescein-dNTPs unter Verwendung des Pharmacia AutoRead Kits

### 1. Denaturierung und Annealing (in einem Gefäß)

10 µl Plasmid-DNA (1/3 einer Minipräparation über eine Quiagensäule), 2 µl nicht markierter Sequenzierprimer (5 µmol/l) und 1 µl 1 mol/l NaOH wurden vermischt, 3 Minuten lang bei 65°C erhitzt und bei 37°C inkubiert.

### 2. Markierung

Zu dem obigen Ansatz wurden 1 µl 1 mol/l HCl, 2 µl Annealing-Puffer, 1 µl Markierungsmischung (10 µmol/l markiertes dNTP und jeweils 1 µmol/l der nicht markierten dNTPs) zusammen mit 0,5 µl T7-DNA-Polymerase (3,5 U) gegeben und 10 Minuten lang bei 37°C inkubiert.

### 3. Termination

Der obige Ansatz wurde mit 1 µl Extensionspuffer vermischt. 4 µl Aliquots davon wurden zu 4 µl der jeweiligen A-, C-, G-und T-Mixe (3 µl der jeweiligen Terminationsmischungen und 1 µl DMSO) gegeben und 5 Minuten lang bei 37°C inkubiert. Die Reaktionen wurden mit 4 µl Stoplösung gestoppt. Anschließend wurde der Ansatz durch Erhitzen denaturiert und auf das Sequenzgel gegeben.

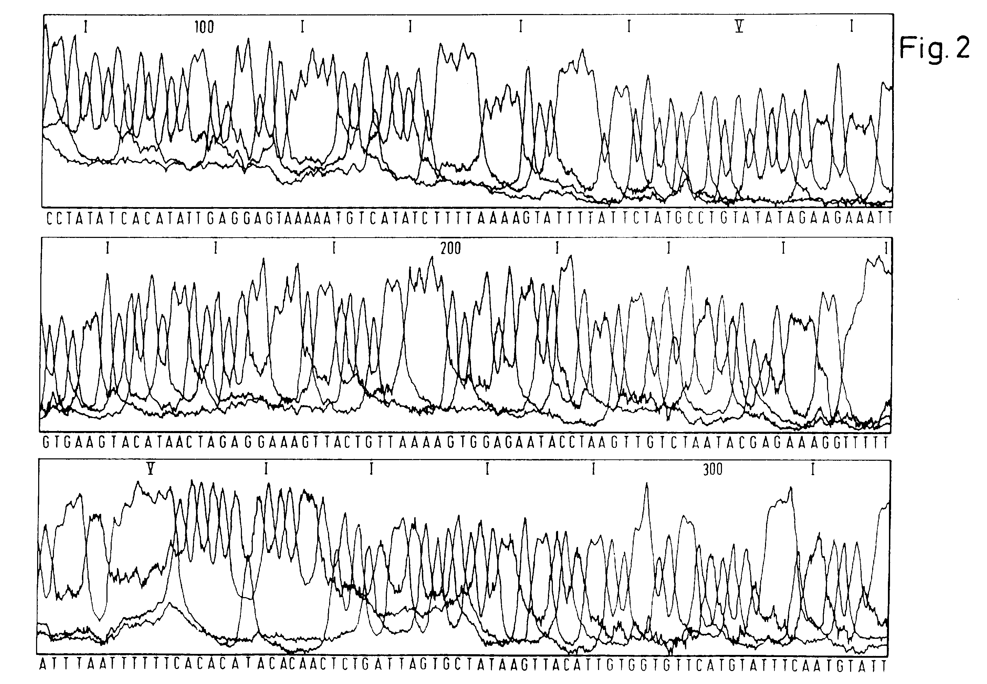

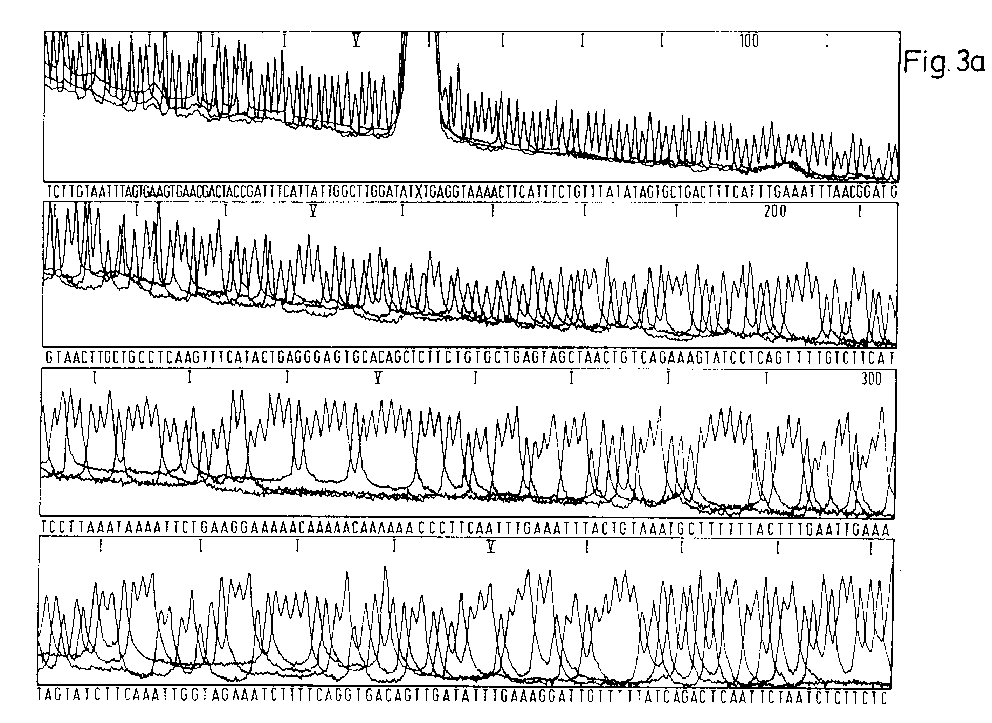

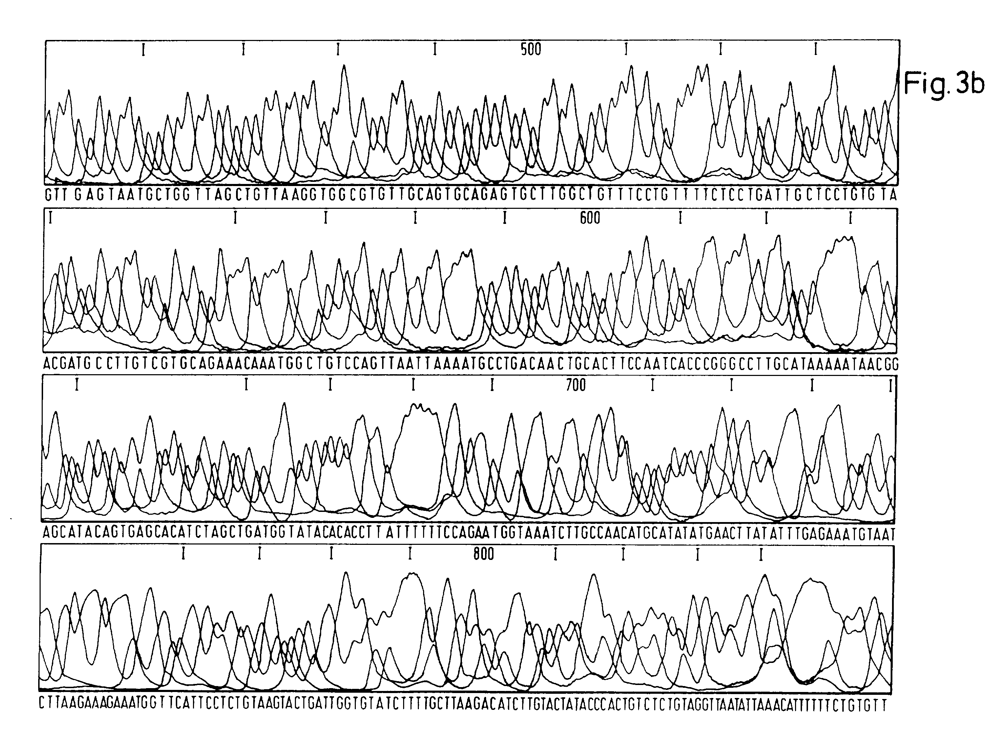

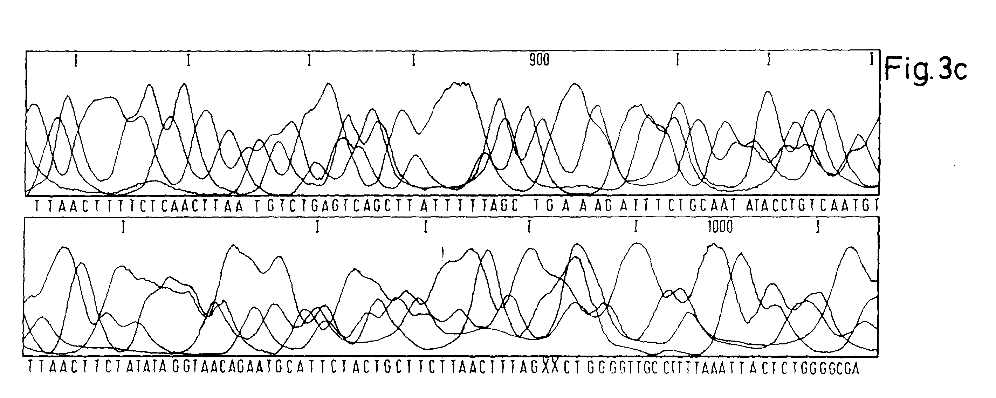

Die Figuren 2 und 3 zeigen die Ergebnisse einer Plasmidsequenzierung unter Verwendung des obigen Protokolls und Fluorescein-12-dCTP (Figur 2) bzw. Fluorescein-15-dATP (Figur 3) als Markierung.

Die Auswertung der Sequenzgele erfolgte auf dem automatischen EMBL Fluorescenc Sequencer. Bei Verwendung von Fluorescein-15-dATP als Markierung ist es möglich, bis zu 1000 Basen zu lesen.

## Patentansprüche

1. Verfahren zur Sequenzierung von Nukleinsäuren durch
(a) Erzeugung eines Gemisches von markierten Nukleinsäurefragmenten mit unterschiedlicher Länge,
(b) Auftrennung der markierten Nukleinsäurefragmente nach Größe und
(c) Bestimmung der Nukleinsäuresequenz über die Markierung der einzelnen Fragmente,
**dadurch gekennzeichnet,**
daß man Nukleinsäurefragmente bestimmt, in die durch Einbau mit mindestens einem Deoxyribonukleosidtriphosphat, das eine nicht-radioaktive Markierungsgruppe trägt, eine innere Markierunq eingeführt worden ist, wobei die Einführung der inneren Markierung in die Nukleinsäurefragmente in Abwesenheit von Kettenabbruchmolekülen stattfindet.

2. Verfahren nach Anspruch 1,
**dadurch gekennzeichnet,**
daß man das Gemisch von markierten Nukleinsäurefragmenten durch die chemische Abbaumethode erzeugt.

3. Verfahren nach Anspruch 2,
**dadurch gekennzeichnet,**
daß man die Markierung durch enzymatische Extension eines Oligonukleotidprimers in Gegenwart einer Polymerase, der zu sequenzierenden Nukleinsäure als Matrize und mindestens einem markierten Deoxyribonukleotid einführt und anschließend mittels chemischer Methoden eine partielle basenspezifische Spaltung der markierten Nukleinsäuren bewirkt.

4. Verfahren nach Anspruch 1,
**dadurch gekennzeichnet,**
daß man das Gemisch von markierten Nukleinsäurefragmenten durch die enzymatische Kettenabbruchmethode erzeugt.

5. Verfahren nach Anspruch 4,
**dadurch gekennzeichnet,**
daß die Einführung der inneren Markierung durch enzymatische Extension eines Oligonukleotidprimers in Gegenwart von Polymerase und Deoxyribonukleosidtriphosphaten erfolgt und anschließend eine Termination der Extensionsreaktion durch Zugabe von Kettenabbruchmolekülen stattfindet.

6. Verfahren nach einem der Ansprüche 1 bis 5,
**dadurch gekennzeichnet**,
daß man Deoxyribonukleosidtriphosphate verwendet, die über einen Linker mit der Markierungsgruppe verknüpft sind.

7. Verfahren nach Anspruch 6,
**dadurch gekennzeichnet,**
daß die Anzahl der Atome in der Kette des Linkers 10 bis 18 beträgt.

8. Verfahren nach einem der Ansprüche 1 bis 7,
**dadurch gekennzeichnet,**
daß man mit Fluoreszenzfarbstoffen markierte Deoxyribonukleosidtriphosphate verwendet.

9. Verfahren nach Anspruch 8,
**dadurch gekennzeichnet,**
daß man als Fluoreszenzfarbstoff Fluorescein oder Derivate davon verwendet.

10. Verfahren nach einem der Ansprüche 1 bis 9,
**dadurch gekennzeichnet,**
daß man als markierte Deoxyribonukleotide Fluorescein-12-dUTP, Fluorescein-12-dCTP, Fluorescein-15-dGTP oder/und Fluorescein-15-dATP verwendet.

11. Verfahren nach Anspruch 3 oder 5,
**dadurch gekennzeichnet,**
daß man bei der enzymatischen Extension einen 5'-unmodifizierten Oligonukleotidprimer verwendet.

12. Verfahren nach Anspruch 3 oder 5,
**dadurch gekennzeichnet,**
daß man einen Oligonukleotidprimer verwendet, der an seinem 5'-Ende mit einem Farbstoff oder/und einer zu einer Affinitätsbindung fähigen Gruppe modifiziert ist.

13. Verfahren nach Anspruch 12,
**dadurch gekennzeichnet,**
daß man einen 5'-biotinylierten Oligonukleotidprimer verwendet.

14. Verfahren nach einem der Ansprüche 4 bis 13,
**dadurch gekennzeichnet**, daß man als Kettenabbruchmoleküle 3'-modifizierte Deoxyribonukleosidtriphosphate verwendet, die an der 3'-Position des Zuckers eine F-, O-Alkyl- oder H-Gruppe aufweisen.

15. Verfahren nach Anspruch 14,
**dadurch gekennzeichnet,**
daß man als Kettenabbruchmoleküle Dideoxyribonukleosidtriphosphate verwendet.

16. Verfahren nach einem der Ansprüche 4 bis 15,
**dadurch gekennzeichnet,**
daß man unmarkierte Kettenabbruchmoleküle verwendet.

17. Verfahren nach einem der Ansprüche 3 oder 5,
**dadurch gekennzeichnet,**
daß man als Polymerase zur enzymatischen Extension das Klenow-Fragment der E.coli DNA-Polymerase I, modifizierte oder unmodifizierte T7-DNA-Polymerase, T4-DNA-Polymerase, Taq-DNA-Polymerase, Bst-DNA-Polymerase oder Reverse Transkriptase verwendet.

18. Verfahren nach einem der Ansprüche 1 bis 17,
**dadurch gekennzeichnet,**
daß die Auftrennung der markierten Nukleinsäurefragmente durch Gelelektrophorese erfolgt.

19. Verfahren nach einem der Ansprüche 1 bis 18,
**dadurch gekennzeichnet,**
daß man die Auftrennung der markierten Nukleinsäurefragmente in einem automatischen Sequenziergerät durchführt.

20. Verfahren nach Anspruch 18 oder 19,
**dadurch gekennzeichnet,**
daß man die Auftrennung in ultradünnen Plattengelen oder Kapillaren durchführt.

21. Verfahren nach einem der Ansprüche 1 bis 20,
**dadurch gekennzeichnet**,
daß man bei Verwendung von mit Fluoreszenzfarbstoffen markierten Deoxyribonukleosidtriphosphaten nach Auftrennung der Nukleinsäurefragmente zur Bestimmung der Markierung den Fluoreszenzfarbstoff durch Laser anregt.

22. Verfahren nach einem der Ansprüche 1 bis 21,
**dadurch gekennzeichnet,**
daß man vor der Sequenzierungsreaktion zur Amplifizierung der Menge der zu sequenzierenden Nukleinsäure einen oder mehrere Zyklen einer PCR-Reaktion durchführt.

23. Verfahren nach einem der Ansprüche 4 bis 22,
**dadurch gekennzeichnet,**
daß man die Sequenzierungsreaktion zur Amplifizierung der Menge der markierten Nukleinsäurefragmente in mehreren Schritten als "Thermocycling"-Reaktion durchführt.

24. Verfahren nach einem der Ansprüche 1 bis 23,
**dadurch gekennzeichnet,**
daß sich die zu sequenzierende Nukleinsäure auf einem doppelsträngigen DNA-Vektor befindet.

25. Verfahren nach Anspruch 23,
**dadurch gekennzeichnet,**
daß der doppelsträngige DNA-Vektor ein Plasmid, Cosmid, Bakteriophage, viraler Vektor oder ein künstliches Chromosom ist.

26. Verfahren nach einem der Ansprüche 5 bis 25,
**dadurch gekennzeichnet**,
daß man die enzymatische Extension bei der Kettenabbruchmethode in mehreren getrennten Ansätzen durchführt, wobei man in jedem Ansatz ein unterschiedliches Kettenabbruchmolekül verwendet.

27. Verfahren nach Anspruch 26,
**dadurch gekennzeichnet,**
daß man in allen Ansätzen zur gleichen Markierung ein oder mehrere gleich markierte Deoxyribonukleosidtriphosphate verwendet, so daß eine getrennte Auftrennung er einzelnen Ansätze erforderlich ist.

28. Verfahren nach Anspruch 26,
**dadurch gekennzeichnet,**
daß man in verschiedenen Ansätzen jeweils unterschiedliche Markierungen verwendet, so daß eine gemeinsame Auftrennung dieser unterschiedlich markierten Ansätze möglich ist.

29. Verfahren nach einem der Ansprüche 4 bis 25,
**dadurch gekennzeichnet,**
daß man mindestens zwei der Kettenabbruchmoleküle in unterschiedlichen Mengen zusammen in einem Ansatz einsetzt und die Unterscheidung der Fragmente über die Intensität ihres Markierungssignals trifft.

30. Verwendung von Deoxyribonukleosidtriphosphaten, die eine nicht-radioaktive Markierung tragen, zur inneren Markierung von Nukleinsäurefragmenten bei einem Verfahren nach einem der Ansprüche 1 bis 29 zur Sequenzierung von Nukleinsäuren, wobei die Einführung der inneren Markierung in die Nukleinsäurefragmente in Abwesenheit von Kettenabbruchmolekülen stattfindet.

31. Verwendung nach Anspruch 30,
**dadurch gekennzeichnet**,
daß die Markierung ein Fluoreszenzfarbstoff ist.

32. Verwendung nach Anspruch 31,
**dadurch gekennzeichnet**,
daß die Markierung Fluorescein oder ein Derivat davon ist.

33. Verwendung nach einem der Ansprüche 31 bis 33,
**dadurch gekennzeichnet,**
daß die Markierungsgruppe mit einem Deoxyribonukleosidtriphosphat über einen Linker verknüpft ist.

34. Verwendung nach Anspruch 33,
**dadurch gekennzeichnet,**
daß die Anzahl der Atome in der Kette des Linkers 10 bis 18 beträgt.

35. Verwendung nach einem der Ansprüche 30 bis 34,
**dadurch gekennzeichnet**,
daß man als markierte Deoxyribonukleosidtriphosphate Fluorescein-12-dUTP, Fluorescein-12-dCTP, Fluorescein-15-dATP oder/und Fluorescein-15-dGTP einsetzt.

36. Verwendung eines Reagenzienkits in einem Verfahren zur Sequenzierung von Nukleinsäuren nach einem der Ansprüche 1 bis 29,
**dadurch gekennzeichnet,**
daß der Kit als separate Reagenzien mindestens ein Deoxyribonukleosidtriphosphat enthält, das mit einer nicht-radioaktiven Markierungsgruppe markiert ist, sowie ferner ein Kettenabbruchmolekül.

## Claims

1. Method for sequencing nucleic acids by
(a) producing a mixture of labelled nucleic acid fragments of different length,
(b) separation of the labelled nucleic acid fragments according to size and
(c) determination of the nucleic acid sequence by means of the labelling of the individual fragments,
**wherein**
nucleic acid fragments are determined into which an inner label has been introduced by incorporation of at least one deoxyribonucleoside triphosphate which carries a non-radioactive labelling group and the inner label is introduced into the nucleic acid fragments in the absence of chain terminating molecules.

2. Method as claimed in claim 1,
**wherein**
the mixture of labelled nucleic acid fragments is produced by the chemical degradation method.

3. Method as claimed in claim 2,
**wherein**
the label is introduced by enzymatic extension of an oligonucleotide primer in the presence of a polymerase, the nucleic acid to be sequenced as a template and at least one labelled deoxyribonucleotide and subsequently a partial base-specific cleavage of the labelled nucleic acids is carried out by means of chemical methods.

4. Method as claimed in claim 1,
**wherein**
the mixture of labelled nucleic acid fragments is produced by the enzymatic chain termination method.

5. Method as claimed in claim 4,
**wherein**
the inner label is introduced by enzymatic extension of an oligonucleotide primer in the presence of polymerase and deoxyribonucleoside triphosphates and subsequently the extension reaction is terminated by addition of chain terminating molecules.

6. Method as claimed in one of the claims 1 to 5,
**wherein**
deoxyribonucleoside triphosphates are used which are linked via a linker to the labelling group.

7. Method as claimed in claim 6,
**wherein**
the number of atoms in the chain of the linker is 10 to 18.

8. Method as claimed in one of the claims 1 to 7,
**wherein**
deoxyribonucleoside triphosphates labelled with fluorescent dyes are used

9. Method as claimed in claim 8,
**wherein**
fluorescein or derivatives thereof are used as the fluorescent dye.

10. Method as claimed in one of the claims 1 to 9,
**wherein**
fluorescein-12-dUTP, fluorescein-12-dCTP, fluorescein-15-dGTP or/and fluorescein-15-dATP are used as labelled deoxyribonucleotides.

11. Method as claimed in claim 3 or 5,
**wherein**
a 5' unmodified oligonucleotide primer is used in the enzymatic extension.

12. Method as claimed in claim 3 or 5,
**wherein**
an oligonucleotide primer is used which is modified at its 5' end with a dye or/and a group capable of affinity binding.

13. Method as claimed in claim 12,
**wherein**
a 5'-biotinylated oligonucleotide primer is used.

14. Method as claimed in one of the claims 4 to 13,
**wherein**
3' modified deoxyribonucleoside triphosphates are used as chain terminating molecules which have a F, O-alkyl, or H group at the 3' position of the sugar.

15. Method as claimed in claim 14,
**wherein**
dideoxyribonucleoside triphosphates are used as chain terminating molecules.

16. Method as claimed in one of the claims 4 to 15,
**wherein**
unlabelled chain terminating molecules are used.

17. Method as claimed in one of the claims 3 or 5,
**wherein**
the Klenow fragment of E. coli DNA polymerase I, modified or unmodified T7 DNA polymerase, T4 DNA polymerase, Taq DNA polymerase, Bst DNA polymerase or reverse transcriptase are used as the polymerase for enzymatic extension.

18. Method as claimed in one of the claims 1 to 17,
**wherein**
the separation of the labelled nucleic acid fragments is achieved by gel electrophoresis.

19. Method as claimed in one of the claims 1 to 18,
**wherein**
the separation of the labelled nucleic acid fragments is carried out in an automatic sequencing instrument.

20. Method as claimed in claim 18 or 19,
**wherein**
the separation is carried out in ultrathin plate gels or capillaries.

21. Method as claimed in one of the claims 1 to 20,
**wherein**
when using deoxyribonucleoside triphosphates labelled with fluorescent dyes, after separating the nucleic acid fragments the fluorescent dye is excited by laser in order to determine the label.

22. Method as claimed in one of the claims 1 to 21,
**wherein**
before the sequencing reaction, one or several cycles of a PCR reaction are carried out to amplify the amount of the nucleic acid to be sequenced.

23. Method as claimed in one of the claims 4 to 22,
**wherein**
the sequencing reaction is carried out in several steps as a "thermocycling" reaction in order to amplify the amount of labelled nucleic acid fragments.

24. Method as claimed in one of the claims 1 to 23,
**wherein**
the nucleic acid to be sequenced is located on a double-stranded DNA vector.

25. Method as claimed in claim 23,
**wherein**
the double-stranded DNA vector is a plasmid, cosmid, bacteriophage, viral vector or an artificial chromosome.

26. Method as claimed in one of the claims 5 to 25,
**wherein**
the enzymatic extension in the chain termination method is carried out in several separate mixtures in which a different chain terminating molecule is used in each mixture.

27. Method as claimed in claim 26,
**wherein**
one or several identically labelled deoxyribonucleoside triphosphates are used for uniform labelling in all mixtures so that a separate separation of the individual mixtures is necessary.

28. Method as claimed in claim 26,
**wherein**
labels that are different in each case are used in different mixtures so that a joint separation of these differently labelled mixtures is possible.

29. Method as claimed in one of the claims 4 to 25,
**wherein**
at least two of the chain terminating molecules are used together in different amounts in one mixture and the fragments are differentiated by means of the intensity of the signal of their label.

30. Use of deoxyribonucleoside triphosphates which carry a non-radioactive label for the inner labelling of nucleic acid fragments in a method as claimed in one of the claims 1 to 29 for sequencing nucleic acids, wherein the inner label is introduced into the nucleic acid fragments in the absence of chain terminating molecules.

31. Use as claimed in claim 30,
**wherein**
the label is a fluorescent dye.

32. Use as claimed in claim 31,
**wherein**
the label is fluorescein or a derivative thereof.

33. Use as claimed in one of the claims 31 to 33,
**wherein**
the labelling group is linked via a linker to the deoxyribonucleoside triphosphate.

34. Use as claimed in claim 33,
**wherein**
the number of atoms in the chain of the linker is 10 to 18.

35. Use as claimed in one of the claims 30 to 34,
**wherein**
fluorescein-12-dUTP, fluorescein-12-dCTP, fluorescein-15-dATP or/and fluorescein-15-dGTP are used as labelled deoxyribonucleoside triphosphates.

36. Use of a reagent kit in a method for sequencing nucleic acids as claimed in one of the claims 1 to 29,
**wherein**
the kit contains as separate reagents at least one deoxyribonucleoside triphosphate which is labelled with a non-radioactive labelling group as well as additionally a chain terminating molecule.

## Revendications

1. Procédé de séquençage d'acides nucléiques, selon lequel
(a) on produit un mélange de fragments d'acides nucléiques marqués de différentes longueurs,
(b) on sépare selon la taille les fragments d'acides nucléiques marqués,
(c) on détermine la séquence des acides nucléiques par l'intermédiaire du marqueur des différents fragments,
caractérisé en ce que
on dose les fragments d'acides nucléiques dans lesquels on a introduit un marqueur interne en incorporant au moins un triphosphate de désoxyribonucléoside portant un groupe de marquage non radioactif, l'introduction du marqueur interne dans les fragments d'acides nucléiques s'effectuant en l'absence de molécules de rupture de chaîne.

2. Procédé selon la revendication 1, caractérisé en ce que l'on produit le mélange de fragments d'acides nucléiques marqués par la méthode de dégradation chimique.

3. Procédé selon la revendication 2, caractérisé en ce que l'on introduit le marqueur par extension enzymatique d'une amorce oligonucléotidique en présence d'une polymérase, de l'acide nucléique à séquencer comme matrice et d'au moins un désoxyribonucléotide marqué, puis on effectue par une méthode chimique une coupure partielle spécifique de bases des acides nucléiques marqués.

4. Procédé selon la revendication 1, caractérisé en ce que l'on produit le mélange de fragments d'acides nucléiques marqués par la méthode de rupture enzymatique des chaînes.

5. Procédé selon la revendication 4, caractérisé en ce que l'introduction du marqueur interne s'effectue par extension enzymatique d'une amorce oligonucléotidique en présence d'une polymérase et de triphosphates de désoxyribonucléosides, et la terminaison de la réaction d'extension se produit ensuite par addition de molécules de rupture de chaîne.

6. Procédé selon l'une des revendications 1 à 5, caractérisé en ce que l'on utilise des triphosphates de désoxyribonucléosides qui sont liés au groupe de marquage par l'intermédiaire d'un lieur.

7. Procédé selon la revendication 6, caractérisé en ce que le nombre d'atomes de la chaîne du lieur est de 10 à 18.

8. Procédé selon l'une des revendications 1 à 7, caractérisé en ce que l'on utilise des triphosphates de désoxyribonucléosides marqués avec des colorants fluorescents.

9. Procédé selon la revendication 8, caractérisé en ce que l'on utilise comme colorant fluorescent la fluorescéine ou ses dérivés.

10. Procédé selon l'une des revendications 1 à 9, caractérisé en ce que l'on utilise comme désoxyribonucléosides marqués le fluorescéine-12-dUTP, le fluorescéine-12-dCTP, le fluorescéine-15-dGTP ou/et le fluorescéine-15-dATP.

11. Procédé selon la revendication 3 ou 5, caractérisé en ce que l'on utilise pour l'extension enzymatique une amorce oligonucléotidique non modifiée en 5'.

12. Procédé selon la revendication 3 ou 5, caractérisé en ce que l'on utilise une amorce oligonucléotidique qui est modifiée à l'extrémité 5' avec un colorant et/ou un groupe capable de former une liaison d'affinité.

13. Procédé selon la revendication 12, caractérisé en ce que l'on utilise une amorce oligonucléotidique biotinylée en 5'.

14. Procédé selon l'une des revendications 4 à 13, caractérisé en ce que l'on utilise comme molécules de rupture de chaîne des triphosphates de désoxyribonucléosides modifiés en 3', qui ont en position 3' du sucre un groupe F, O-alkyle ou H.

15. Procédé selon la revendication 14, caractérisé en ce que l'on utilise comme molécules de rupture de chaînes des triphosphates de didésoxyribonucléosides.

16. Procédé selon l'une des revendications 4 à 15, caractérisé en ce que l'on utilise des molécules de rupture de chaîne non marquées.

17. Procédé selon l'une des revendications 3 ou 5, caractérisé en ce que l'on utilise comme polymérase pour l'extension enzymatique le fragment de Klenow de l'ADN-polymérase I d'*E*. *coli,* l'ADN-polymérase de T7 modifiée ou non modifiée, l'ADN-polymérase de T4, l'ADN-polymérase de Taq, l'ADN-polymérase de Bst ou une transcriptase inverse.

18. Procédé selon l'une des revendications 1 à 17, caractérisé en ce que la séparation des fragments d'acides nucléiques marqués s'effectue par électrophorèse en gel.

19. Procédé selon l'une des revendications 1 à 18, caractérisé en ce que l'on effectue la séparation des fragments d'acides nucléiques marqués dans un appareil de séquençage automatique.

20. Procédé selon la revendication 18 ou 19, caractérisé en ce que l'on effectue la séparation dans des gels de plaques ultraminces ou des capillaires.

21. Procédé selon l'une des revendications 1 à 20, caractérisé en ce que, lorsque l'on utilise des triphosphates de désoxyribonucléosides marqués avec des colorants fluorescents, après la séparation des fragments d'acides nucléiques, on excite le colorant fluorescent au laser pour doser le marqueur.

22. Procédé selon l'une des revendications 1 à 21, caractérisé en ce que l'on effectue avant la réaction de séquençage un ou plusieurs cycles de réaction de PCR pour amplifier la quantité de l'acide nucléique à séquencer.

23. Procédé selon l'une des revendications 4 à 22, caractérisé en ce que l'on effectue la réaction de séquençage en plusieurs étapes sous forme d'une réaction de "thermocycling", pour amplifier la quantité des fragments d'acides nucléiques marqués.

24. Procédé selon l'une des revendications 1 à 23, caractérisé en ce que l'acide nucléique à séquencer se trouve sur un vecteur d'ADN double brin.

25. Procédé selon la revendication 23, caractérisé en ce que le vecteur d'ADN double brin est un plasmide, un cosmide, un bactériophage, un vecteur viral ou un chromosome synthétique.

26. Procédé selon l'une des revendications 5 à 25, caractérisé en ce que l'on effectue l'extension enzymatique dans la méthode de rupture des chaînes en plusieurs charges séparées, en utilisant dans chaque charge une molécule de rupture de chaîne différente.

27. Procédé selon la revendication 26, caractérisé en ce que l'on utilise dans toutes les charges, pour le même marquage, un ou plusieurs triphosphates de désoxyribonucléosides marqués de la même manière, de sorte qu'une séparation séparée des différentes charges est nécessaire.

28. Procédé selon la revendication 26, dans lequel on utilise des marquages différents pour les différentes charges, de sorte que l'on peut séparer en même temps ces charges marquées différemment.

29. Procédé selon l'une des revendications 4 à 25, caractérisé en ce que l'on utilise ensemble au moins deux des molécules de rupture de chaîne en des quantités différentes dans une charge, et on différencie les fragments par l'intensité du signal de leur marqueur.

30. Utilisation de triphosphates de désoxyribonucléosides portant un marqueur non radioactif pour le marquage inteme de fragments d'acides nucléiques dans un procédé selon l'une des revendications 1 à 29 pour le séquençage d'acides nucléiques, l'introduction du marqueur interne dans les fragments d'acides nucléiques s'effectuant en l'absence de molécules de rupture de chaîne.

31. Utilisation selon la revendication 30, caractérisée en ce que le marqueur est un colorant fluorescent.

32. Utilisation selon la revendication 31, caractérisée en ce que le marqueur est la fluorescéine ou un de ses dérivés.

33. Utilisation selon l'une des revendications 31 à 32, caractérisée en ce que le groupe marqueur est lié à un triphosphate de désoxyribonucléoside par l'intermédiaire d'un lieur.

34. Utilisation selon la revendication 33, caractérisée en ce que le nombre des atomes dans la chaîne du lieur est de 10 à 18.

35. Utilisation selon l'une des revendications 30 à 34, caractérisée en ce que l'on utilise comme triphosphates de désoxyribonucléosides marqués le fluorescéine-12-dUTP, le fluorescéine-12-dCTP, le fluorescéine-15-dATP ou/et le fluorescéine-15-dGTP.

36. Utilisation d'une trousse de réactifs dans un procédé de séquençage d'acides nucléiques selon l'une des revendications 1 à 29, caractérisée en ce que la trousse contient comme réactifs séparés au moins un triphosphate de désoxyribonucléoside marqué avec un groupe marqueur non radioactif, ainsi qu'une molécule de rupture de chaîne.
